# EUROPEAN PATENT SPECIFICATION

(11) **EP 2 398 823 B1**
(45) Date of publication and mention of the grant of the patent: **23.03.2016**
(21) Application number: 09788100.7
(22) Date of filing: 05.06.2009
(51) Int. Cl.: C07K 16/12, C12N 5/12, A61K 39/40

(54) **MONOCLONAL ANTIBODIES AGAINST VAGINOLYSIN**
MONOKLONALE ANTIKÖRPER GEGEN VAGINOLYSIN
ANTICORPS MONOCLONAUX CONTRE LA VAGONILYSINE

(30) Priority: 23.02.2009 LT 2009012
(43) Date of publication of application: 28.12.2011
(73) Proprietor: UAB Profarma, 02241 Vilnius (LT)
(72) Inventor: ZVIRBLIENE, Aurelija, LT-06111 Vilnius (LT); KUCINSKAITE-KODZE, Indre, LT-07138 Vilnius (LT); PLECKAITYTE, Milda, LT-10228 Vilnius (LT); ZVIRBLIS, Gintautas, LT-06111 Vilnius (LT)
(74) Representative: Gerasimovic, Liudmila
(86) International application number: PCT/LT2009/000005
(87) International publication number: WO 2010/095917

(56) References cited:
- WO-A-2009/117373
- GELBER SHARI E ET AL: "Functional and phylogenetic characterization of vaginolysin, the human-specific cytolysin from Gardnerella vaginafis" JOURNAL OF BACTERIOLOGY, vol. 190, no. 11, June 2008 (2008-06), pages 3896-3903, XP009125383 ISSN: 0021-9193
- RANDIS TARA M ET AL: "Antibody-Based Detection and Inhibition of Vaginolysin, the Gardnerella vaginalis Cytolysin" PLOS ONE, vol. 4, no. 4, April 2009 (2009-04), page Article No.: e5207, XP009128317 ISSN: 1932-6203
- EDELSON BRIAN T ET AL: "Intracellular antibody neutralizes Listeria growth" IMMUNITY, vol. 14, no. 5, May 2001 (2001-05), pages 503-512, XP009127900 ISSN: 1074-7613
- DATABASE UniProt [Online] 1 July 2008 (2008-07-01), "SubName: Full=Cholesterol-dependent cytolysin;" XP002564597 retrieved from EBI accession no. UNIPROT:B2ZUI2 Database accession no. B2ZUI2
- AURELIJA ZVIRBLIENE ET AL: 'Production and characterization of monoclonal antibodies against vaginolysin: Mapping of a region critical for its cytolytic activity' TOXICON vol. 56, no. 1, 01 August 2010, US, pages 19 - 28, XP002648819 DOI: 10.1016/J.TOXICON.2010.03.007 ISSN: 0041-0101

## Description

### Field of the Invention

This invention belongs to the field of biotechnology; actually, it describes the generation of new monoclonal antibodies that neutralize biological activity of vaginolysin.

### Background of the Invention

The cholesterol-dependent cytolysins (CDCs) belong to a large family of pore-forming toxins that are produced by more than 20 species from the genera *Clostridium, Streptococcus, Listeria, Arcanobacterium, Bacillus* and others. These toxins (often called as cytolysins or hemolysins) are associated with dermatological infections (Streptolysin O; Pyolysin), acute fever, rheumatic fever, pharyngitis (Streptolysin O), pulmonary infections (Pneumolysin), listeriosis (Listeriolysin O), contribute to the pathogenesis of various pneumococcal disease syndromes (Pneumolysin), and are involved in the initial stage of tissue damage leading to gas gangrene (Perfingolysin O). All these toxins are believed to bind to cholesterol as receptor in the host cell membranes that subsequently leads to insertion and oligomerization. Consequently, the membrane is permeabilized. However, besides membrane permeabilization these cytolysins exhibit several other functions including induction of cytokine secretion and other modulators of the immune system. Thus, most likely these cytolysins play essential roles as virulence factors in the progression of an infection by said bacteria [Tweten R. K. Infect. Immun. 2005, 73, pp.6199-6209*].*

The most conserved structural domain of toxins belonging to this family is located in the C-terminus of protein and consists of 11 amino acids (undecapeptide). All family members possess this structural element, which is crucial for the cytolytic activity of toxins [Jacobs et al., FEBS Lett. 1999, 459, pp. 463-466]. It is indirectly assumed that *Gardnerella vaginalis* secreted exotoxin (VLY) may have relations with the cited class of cholesterol-dependent cytolysins (CDCs) so that investigations in this area may have strong impact on the development of actual appropriate therapeutic and diagnostic tools against serious infection suffered by women.

Vaginolysin (VLY) is protein toxin, produced by bacteria *Gardnerella vaginalis.* This protein is classified as a member of cholesterol-dependent cytolysin family. Toxic effect of VLY is based on forming pores in the cell membrane causing human cell lysis.
Bacteria *Gardnerella vaginalis* is known as causing agent of bacterial vaginosis (BV) and the secreted toxin VLY is the main virulence factor [Cauci S, Monte M, Ropele C et al., Mol Microbiol, 1993, 9, pp. 53-58]. BV is a common condition, affecting millions of women annually, and is associated with numerous health problems including preterm labour resulting in low birth weight that is the main risk factor for neonatal mortality and morbidity, pelvic inflammatory disease, postpartum endometritis and other infection diseases. It might be supposed that neutralization of VLY using antibodies could be an effective method for a treatment of pathogenic infection induced by *Gardnerella vaginalis.*

Development of protecting monoclonal antibodies against bacterial toxins has been described since 1993. New monoclonal antibodies against tetanus toxin have been developed and analyzed for their protection effect [Lang et el., J. Immunol. 1993, 151, pp. 466-472]. Monoclonal antibodies against botulism toxin have been developed using phage-display libraries, obtained from immunized mouse or human [Brekke and Sandlie, Nat.Rev. Drug Discov. 2002, 2, pp. 52-62]. Recently murine monoclonal antibodies against pneumolysin have been developed and their efficiency was tested on animal models [Garsia-Suarez et al., Infect. Immun. 2004, 72, pp. 4534-4540]. Development of murine monoclonal antibody PLY5 which is neutralizing various cytolysins has been reported. It was shown that this monoclonal antibody is binding to specific epitope - undecapeptide, which is common to cholesterol binding toxin group. This monoclonal antibody inhibits binding of toxins to the cell membrane due to hiding of this undecapeptide epitope [Jacobs et al., FEBS Lett. 1999, 459, pp. 463-466].

Antibodies are widely used as biopharmaceutical preparations due to the progress in gene engineering and humanization technologies. Genes encoding variable fragments of immunoglobulins can be cloned and expressed in *E. coli* or phage-display systems. Maynard et al. reported development of high-affinity single chain antibody fragments (scFv) against *Bacillus anthracis* [Maynard et al., Nature Biotechnol. 2002, 30, pp. 597-601]. By injecting these scFv into mice they were protected from a letal effect of *Bacillus anthracis* toxin. Neutralizing human antibodies against Shiga toxin 1 were produced by using transgenic mice [Mukherjee et al., Infect. Immun. 2002, 70, pp. 5896-5899]. Neutralizing scFv against Scorpio toxin II were developed and characterized [Mousli et.al., FEBS Lett. 1999, 442 pp. 183-188].

Recently, recombinant vaginolysin was produced by using gene engineering approaches. It was used for investigation of action mechanism [Gelber SE, Aguilar JL, Lewis KLT et al., J Bacteriol, 2008, 190, pp. 3896-3903].
Monoclonal antibodies against *Streptococcus pneumoniae* toxin pneumolysin (PLY) were described [EP0687688, publ. 1995; Toyos JR, Mendez FJ, Aparico JF et al., Infect Immun, 1996, 64, pp.480-484; Garcia-Suarez MM, Cima-Cabal MD, Florez N et al., Infect Immun, 2004, 72, pp. 4534-4540]. PLY belongs to CDC family but differs from VLY according to its structure, action mode and other properties. Nine monoclonal antibodies against PLY were developed (PLY-1 - PLY-9). Three of them were neutralizing, i.e, they inhibited the biological activity of PLY both *in vitro* and *in vivo.* In mouse models, protection activity of 2 monoclonal antibodies (PLY-4 and PLY-7) was demonstrated. The analogs of PLY-1 - PLY-9 are monoclonal antibodies 26-5F12, 26-23C2, 22-6E6 against PLY that neutralize hemolytic activity of PLY [WO2006021210 publ. 2006].
Gelber SE publication above is mentioning that anti-PLY monoclonal antibody recognizes recombinant vaginolysin, but is not disclosing inhibiting or neutralizing activity thereof.
Monoclonal antibodies against streptolysin (SLO) were described [WO9305152, publ. 1993].
Monoclonal antibodies against listeriolysin (LLO) from *Listeria monocytogenes* were also described [Edelson TB, Cossart P, Unanue ER, J Immunol, 1999, 163, pp. 4087-4890; Edelson TB, Unanue ER, Immunity, 14, pp. 503-512]. LLO is the main virulence factor of *Listeria monocytogenes* that is forming pores in cellular membranes. Monoclonal antibody A4-8 against LLO was developed that neutralized its biological activity both *in vitro* and *in vivo.* In mouse models, it was demonstrated that antibody A4-8 protected mice from a letal infection of *Listeria monocytogenes.*
The above examples suggest that neutralizing monoclonal antibodies against bacterial toxins may have a potential therapeutic impact for treatment of pathological changes induced by bacterial infections.

Monoclonal antibodies against vaginolysin from *Gardnerella vaginalis* are not found to be described in the prior art. The previuously described analogs were developed against other bacterial toxins that differ both structurally and functionally from vaginolysin, though belong to the same family of pore-forming cytolysins.
There is a need for an efficient mean to detect vaginolysin and neutralize its cytolitic activity and this was the task of the present invention. The problem might be solved by producing new monoclonal antibodies against vaginolysin that should recognize native vaginolysin from *Gardnerella vaginalis* and neutralize its capability to induce cell lysis. These antibodies might be useful for diagnostics and for developing of biopharmaceutical recombinant antibodies with a potential therapeutic use.

### Summary of the Invention

The aim of the present invention was generation of new hybridoma cell lines producing monoclonal antibodies against vaginolysin from *Gardnerella vaginalis.*
The present invention provides two new hybridoma cell lines titled 9B4 and 23A2. These hybridoma cell lines are found to produce monoclonal antibodies against vaginolysin. It is demonstrated that these monoclonal antibodies recognize both recombinant vaginolysin expressed in *E.coli* and native vaginolysin from *Gardnerella vaginalis.* It is also demonstrated in the present invention that these monoclonal antibodies neutralize the biological activity of vaginolysin *in vitro,* i.e., inhibit its capability to induce lysis of human erythrocytes.

The present invention provides a monoclonal antibody that recognizes amino acid sequence SEQ ID: NO.1 or similar sequence, which identity is at least 95%.

The preferred embodiment provides a use of the monoclonal antibody of present invention for producing of preparations for the detection of infection, such as bacterial vaginosis induced by *Gardnerella vaginalis.*

Another preferred embodiments of the present invention provides a diagnostic kit for the detection of *Gardnerella vaginalis* infection, comprising at least a monoclonal antibody according to present invention in separate container(s).

Yet another preferred embodiment provides a use of the hybridoma according to present invention for manufacture of preparations for inhibiting or neutralizing biological activity of vaginolysin and provides a corresponding preparation produced from said hybridomas for inhibiting of infection, such as bacterial vaginosis, induced by *Gardnerella vaginalis.* Such preparations comprising the genetic material from hybridomas of present invention, are suitable for developing biopharmaceutical recombinant antibodies with a potential therapeutic use.

Finally present invention provides a pharmaceutical composition, comprising an effective amount of preparation of present invention in combination with pharmaceutically acceptable carrier, diluent, excipient and/or auxiliary substances for the prophylaxis and/or treatment of conditions induced by *Gardnerella vaginalis* infection. Preferred embodiment of the present invention covers a group of conditions (pathologies), comprising preterm labour resulting in low birth weight, pelvic inflammatory disease, postpartum endometritis and other diseases induced by *Gardnerella vaginalis* infection.

The monoclonal antibody of present invention produced by clone 9B4 shows more potent neutralizing activity as compared to the antibody produced by clone 23A2. The monoclonal antibodies disclosed in the present invention are new and differ from the analogs of the prior art.

The invention is described below in further details and with reference to the accompanying drawings and examples.

### Detailed Description of the Invention

In order to generate new hybridomas, recombinant vaginolysin was employed. The following known to the persons skilled, standard procedures were used:
1) Immunization - recombinant vaginolysin (antigen) was injected subcutaneously into BALB/c mice. Before the injection, antigen solution was mixed with an equal volume of complete Freund's adjuvant.
2) Boost immunization -four weeks later the mice were injected subcutaneously again with the same dose of the antigen mixed with incomplete Freund's adjuvant.
3) Further four weeks later the mice were injected with the same dose of the antigen without an adjuvant. During immunization, the development of antibodies against the antigen in the sera of immunized mice was monitored. The monitoring was performed by a known method - enzyme-linked immunosorbent assay (ELISA).
4) Three days after the 3rd immunization spleen cells were fused with tumor cells, namely, myeloma Sp 2/0 cells, by using polyethylene glycol (PEG) as a fusion agent (Sigma, USA). This and further procedures were performed according to the previously described procedure used to generate hybridomas [Kohler, Milstein, Nature 1975; 256:495-497].
5) Obtained hybrid cell clones (hybridomas) were tested for their specificity, e.g., it was determined what kind of antibodies they produce. An ELISA assay was used for this purpose. The aim of this testing was to determine if the hybridomas produce antibodies against vaginolysin.
6) The hybridomas were characterized as follows:
   i. isotype of produced monoclonal antibodies was determined;
   ii. specificity of monoclonal antibodies was studied by ELISA and Western blotting.
7) The hybridomas that were identified to produce antibodies against vaginolysin were cloned, propagated and frozen for a further storage.
8) Antibodies produced by the hybridomas were characterized by well-known methods:
   i. ELISA and Western blotting using recombinant vaginolysin as an antigen;
   ii. Western blotting using the lysates of *Gardnerella vaginalis;*
   iii. neutralizing activity of the antibodies was studied by using human erythrocytes.
Thus, by immunizing BALB/c mice with recombinant vaginolysin and further fusing spleen cells of the immunized mice with myeloma cells the hybridomas were generated that produce monoclonal antibodies against vaginolysin. It was determined that these monoclonal antibodies recognize native vaginolysin from *Gardnerella vaginalis* and neutralize its cytolytic activity *in vitro.*
By using the above described procedures 2 new hybridoma cell lines were generated, that are deposited as follows:
1) Hybridoma 9B4, previously deposited as VLY001 in Institute of Biotechnology, Vilnius and currently deposited at DSMZ under accession number DSM ACC2991, that produces monoclonal antibody of IgG1 subtype against vaginolysin;
2) Hybridoma 23A2, previously deposited as VLY002 in Institute of Biotechnology, Vilnius and currently deposited at DSMZ under accession number DSM ACC2992, that produces monoclonal antibody of IgG1 subtype against vaginolysin.

Hybridomas 9B4 and 23A2 are characterized by the following features.
*Morphologic properties.* Hybridomas are round-shaped, with a large nucleus. They grow in suspension, are slightly adherent on glass or plastic surface.
*Parent cell cultures.* Hybridomas were obtained by fusion of spleen cells of the immunized BALB/c mouse (*Mus.musculus*) with mouse (*Mus.musculus*) myeloma cell line Sp 2/0.
*Cell culture properties.* Hybridomas are cultivated under standard conditions. Their growth medium is Dulbecco modified Eagl's medium (DMEM) with 2mM L-glutamine, 200 µg/mL gentamicin and 10% fetal calf serum. Hybridomas are cultivated in glass or plastic flasks for cell culture. The cells are subcultivated every 3-4 days, inoculation dose is 50-100 thousands of cells per milliliter of growth medium. Hybridomas are grown in an incubator in 5% CO₂ atmosphere at 37°C. For a long storage, the hybridomas are frozen in fetal calf serum containing 10% DMSO. They are stored in liquid nitrogen. Freezing conditions: 1×10⁶ - 2×10⁶ cells per milliliter, temperature decrease - 1°C/min. until -70°C. After 24 hrs the vials with frozen cells are transferred to liquid nitrogen. For a thawing, the cultures are incubated in a water bath at 37°C. The viability of cells after thawing - 70-80%.
*Contamination.* Hybridoma cultures are free of bacteria and fungi. Mycoplasma test is negative.
*Productivity of hybridomas. Monoclonal* antibody secretion level in growth medium - 10-30 µg/ml. The stability of antibody secretion - up to 15 passages (sub-cultures).
*Characterization of the products*. Hybridomas 9B4 and 23A2 produce monoclonal antibodies of IgG isotype, IgG1 subtype, specific to vaginolysin from *Gardnerella vaginalis* and neutralizing its cytolytic acitvity *in vitro.*
Monoclonal antibody produced by hybridoma 9B4 completely inhibits the cytolytic activity of vaginolysin in the presence of 5 ng/ml of vaginolysin and 20 ng/ml of the antibody. Monoclonal antibody produced by hybridoma 23A2 completely inhibits the cytolytic activity of vaginolysin in the presence of 5 ng/ml of vaginolysin and 200 ng/ml of the antibody Monoclonal antibodies produced by hybridomas 9B4 and 23A2 differ from the analogs described previously as they are specific to vaginolysin (VLY). The VLY is different from other cytolysins both structurally and functionally. VLY differs from other cytolysins by the following features:
1) Protein primary structure (amino acid sequence);
2) Origin - VLY is produced by bacteria *Gardnerella vaginalis;*
3) Biological activity - VLY lyses exclusively human erythrocytes;
4) Recognized cell surface receptors - VLY binds to human erythrocytes via CD59 molecule.
It was found in present invention, that monoclonal antibodies produced by hybridomas 9B4 and 23A2 recognize the following amino acid (aa) sequence, SEQ ID: No.1: This aa sequence corresponds to aa sequence of vaginolysin from *Gardnerella vaginalis* that was determined and published previously (GenBank Nr. EU697812, EU 697811). Different strains of *Gardnerella vaginalis* may express slightly different variants of vaginolysin that may differ from the SEQ ID: No.1 by one or few aa residues.
Previously described monoclonal antibodies PLY-5, PLY-7, 26-5F12, 26-23C2, 22-6E6 - differ from monoclonal antibodies produced by hybridomas 9B4 and 23A2 as they are specific to pneumolysin (PLY) of *Streptococcus pneumoniae* and neutralize its cytolytic activity [EP0687688, WO2006021210]. Previously described monoclonal antibodies against streptolysin (SLO) differ from monoclonal antibodies produced by hybridomas 9B4 and 23A2 as they are specific SLO and variants thereof [WO9305152]. Previously described monoclonal antibody A4-8 differs from monoclonal antibodies produced by hybridomas 9B4 and 23A2 as it is specific to the listeriolysin (LLO) from *Listeria monocytogenes* and neutralizes its cytolytic activity.

### Brief Description of the Drawings:

The generation of new hybridomas and the properties of new monoclonal antibodies are illustrated by Figures 1-3.
Fig.1 shows Western blotting picture of recombinant vaginolysin and the lysates of bacteria *Gardnerella vaginalis* and *E.coli.* The membranes were developed with monoclonal antibodies produced by hybridoma clones 9B4 and 23A2.
Fig.2 shows Western blotting picture of recombinant vaginolysin and cytolysins SLY, LLO and PLY. The membranes were developed with monoclonal antibodies produced by hybridoma clones 9B4 and 23A2.
Fig.3 shows the neutralizing activity of monoclonal antibodies produced by hybridoma clones 9B4 and 23A2.

### Embodiments of the Invention

Represented below is information on specific examples on generation of new monoclonal antibodies and properties thereof. These examples 1-4 and Table 1 are presented for illustrative purpose and are not limiting the scope of the present invention.

### Example 1.

### Generation of hybridoma cell lines producing monoclonal antibodies against vaginolysin from Gardnerella vaginalis.

BALB/c mice were immunized with recombinant vaginolysin (antigen) expressed in *E.coli.* Mice were immunized by injecting subcutaneously 50 µg of the antigen mixed with complete Freund's adjuvant. Four weeks later mice were boosted in a similar way with 50 µg of the antigen in incomplete Freund's adjuvant. Further four weeks later mice were boosted again with 50 µg of the antigen without an adjuvant. During immunization, the titers of antibodies specific to the antigen were tested by an enzyme-linked immunosorbent assay (ELISA). On day 3 after the last boost the hybridization was performed.
Spleen cells of the immunized mice were fused with mouse myeloma Sp 2/0 cells lacking an enzyme hypoxanthine phosphoribosyl transferase, non-proliferating in a selective HAT medium with hypoxanthine-aminopterin-thymidine (10⁻⁴M hypoxanthine, 1,6 × 10⁻⁵M thymidine, 4 × 10⁻⁷M aminopterin), non-producing imunoglobulins nor their chains. Before fusion, the cells should be in a logarithmic growth phase. The fusion was performed by incubation the cells for 2 min with 50% polyethylene glycol solution (PEG-4000) in Dulbecco modified Eagl's medium (DMEM) with 10% dimethylsulfoxide (DMSO). The ratio of myeloma and spleen cells during fusion was 1:5. After fusion, the cells were washed with serum-free DMEM, resuspended in a selective HAT medium and seeded into multiwell plates at a density 5×10⁵ cells per well. Hybrid clones appeared on days 5-10 after fusion. Growth medium was tested by an indirect ELISA for the antibodies specific to vaginolysin. Every 4-5 days one-half of the growth medium was changed. For a first time HAT medium was changed to HT medium containing hypoxanthine-thymidine (10⁻⁴ M hypoxanthine and 1,6 × 10⁻⁴ M thymidine). Later on, HT medium was changed stepwise to normal growth medium. Hybrid clones producing antibodies specific to vaginolysin were cloned by limiting dilution assay. The clones appeared on days 4-7 after cloning. They were tested by an indirect ELISA. Selected clones were propagated and then either cultivated *in vitro,* or frozen for a storage in liquid nitrogen. Several hybridoma clones producing monoclonal antibodies against vaginolysin were obtained. The specificity, isotypes and neutralizing activity of monoclonal antibodies were tested. Two hybridoma cell lines producing neutralizing monoclonal antibodies against vaginolysin were designed 9B4 and 23A2. They were deposited in the collection of the Institute of Biotechnology (Vilnius) under the deposit numbers:
- hybridoma 9B4 - VLY001, currently deposited at the DSMZ under the respective accession number DSM ACC2991, and
- hybridoma 23A2 - VLY002, currently deposited at DSMZ under the respective accession number DSM ACC2992.
Date of deposition of hybridomas mentioned at DSMZ, the recognised collection in accordance with Budapest Treaty, is 22-04-2009.

### Example 2.

### Determination of the specificity and isotypes of monoclonal antibodies

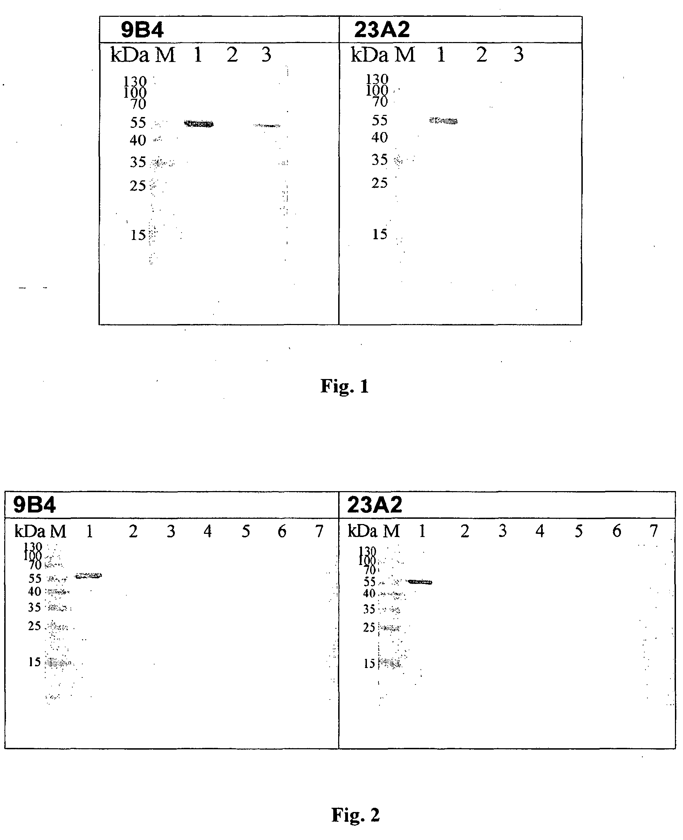

The specificity of antibodies produced by clones 9B4 and 23A2 was determined by ELISA and Western blotting. Imunoglobulin isotypes were determined by ELISA using antibody isotyping kit [Sigma, USA].
It was determined by using these assays that monoclonal antibodies are of IgG isotype, IgG1 subtype and recognize recombinant vaginolysin (Fig. 1). By using Western blotting it was demonstrated that monoclonal antibodies react with 57 kDa protein in the lysates of *Gardnerella vaginalis* that corresponds to vaginolysin (VLY) according to its molecular weight (MW). In contrast, they do not react with bacterial proteins in *E.coli* lysates (Fig. 1). Therefore, monoclonal antibodies obtained are specific to vaginolysin from *Gardnerella vaginalis.*

The data on the specificity and isotypes of monoclonal antibodies produced by hybridomas 9B4 and 23A2 are summarized in Table 1.

**Table 1**

| Hybridoma clone | Subtype (subclass) | Reactivity with: | | |
|---|---|---|---|---|
| | | Recombinant VLY | *Gardnerella vaginalis* lysate | *E.coli* lysate |
| 9B4 | IgG1 | + | + | - |
| 23A2 | IgG1 | + | + | - |

Fig. 1 shows Western blotting picture where recombinant VLY and lysates of bacteria *Gardnerella vaginalis* and *E.coli* were immunostained with monoclonal antibodies produced by hybridomas 9B4 and 23A2. Monoclonal antibody codes are indicated on the top of each picture.
Lane 1 - recombinant purified VLY;
lane 2 - *E.coli* lysate;
lane 3 - *Gardnerella vaginalis* lysate;
M - prestained MW marker.

### Example 3.

### Investigation of the cross-specificity of monoclonal antibodies

By using Western blotting the monoclonal antibodies were tested for their cross-specificity, i.e., their reactivity with recombinant cytolysins SLY, LLO and PLY. Recombinant cytolysins expressed in *E.coli* were used for this investigation. As a positive control, recombinant VLY was used. As a negative control, *E.coli* lysate was used.
This investigation has shown that monoclonal antibodies react exclusively with VLY and do not react with other recombinant cytolysins - SLY, LLO, PLY (Fig. 2) Thus, monoclonal antibodies are specific to vaginolysin from *Gardnerella vaginalis* and differ from previously described analogs.
Fig. 2 shows Western blotting picture where recombinant VLY and recombinant-cytolysins SLY, LLO, PLY were immunostained with monoclonal antibodies produced by hybridomas 9B4 and 23A2. Monoclonal antibody codes are indicated on the top of each picture.
Lane 1 - recombinant purified VLY;
lane 2 - *E.coli* lysate;
lane 3 - *E.coli* lysate with SLY;
lane 4 - *E.coli* lysate;
lane 5 - *E.coli* lysate with LLO;
lane 6 - *E.coli* lysate;
lane 7 - *E.coli* lysate with PLY;
M - prestained MW marker.

### Example 4.

### Investigation of the neutralizing activity of monoclonal antibodies

The ability of antibodies produced by clones 9B4 and 23A2 to neutralize cytolytic activity of VLY was investigated by using known assay [Walker JA, Allen RL, Falmagne P et al., Infect Immun, 1987, 55, pp. 1184-1189; Cauci S, Monte R, Ropele M et al., Mol Microbiol, 1993, 9, pp. 1143-1155.]. The suspension of human erythrocytes (1 ml) was incubated for 15 min at 22°C in the following reaction mixtures:
1) Phosphate-buffered saline (PBS), pH 7,4 (0% hemolysis);
2) Deionized water (100% hemolysis);
3) PBS with 5 ng/ml VLY (100% hemolysis);
4) PBS with 5 ng/ml VLY and 20 ng/ml antibody 9B4;
5) PBS with 5 ng/ml VLY and 200 ng/ml antibody 9B4;
6) PBS with 5 ng/ml VLY and 1000 ng/ml antibody 9B4;
7) PBS with 5 ng/ml VLY and 20 ng/ml antibody 23A2;
8) PBS with 5 ng/ml VLY and 200 ng/ml antibody 23A2;
9) PBS with 5 ng/ml VLY and 1000 ng/ml antibody 23A2.

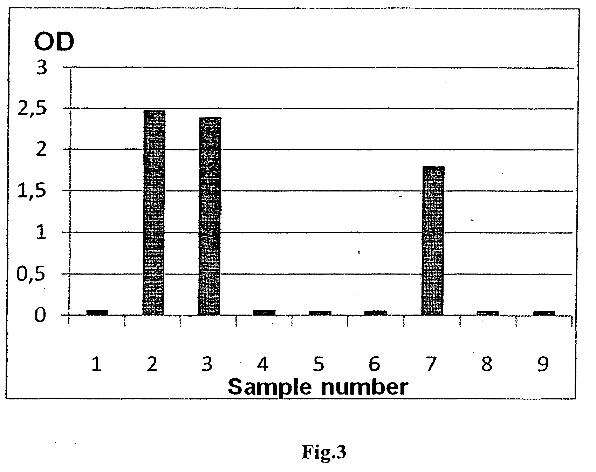

After incubation, erythrocyte suspension was centrifuged (3 min, 1000xg) and the optical density (OD) was measured at a wavelength of 415 nm. High OD values (OD>2.0) indicate hemolysis. Low OD values (OD<0.2) indicate that the erythrocytes were protected from lysis. In the presence of 5 ng/ml of recombinant VLY, the erythrocytes were completely lysed (OD = 2.5). In the presence of either 20 ng/ml of antibody 9B4 or 200 ng/ml antibody 23A2, the erythrocytes were completely protected from lysis (Fig.3). This indicates that both antibodies were capable to neutralize cytolytic activity of VLY. Antibody 9B4 was more potent than 23A2 as it neutralized VLY at lower concentrations.
Fig.3 shows neutralizing activity of monoclonal antibodies produced by clones 9B4 and 23A2 . At Y axis, OD values are shown. At X axis, the numbers of the following reaction mixtures are indicated:
1) Phosphate-buffered saline (PBS);
2) Deionized water;
3) PBS with 5 ng/ml VLY;
4) PBS with 5 ng/ml VLY and 20 ng/ml antibody 9B4;
5) PBS with 5 ng/ml VLY and 200 ng/ml antibody 9B4;
6) PBS with 5 ng/ml VLY and 1000 ng/ml antibody 9B4;
7) PBS with 5 ng/ml VLY and 20 ng/ml antibody 23A2;
8) PBS with 5 ng/ml VLY and 200 ng/ml antibody 23A2;
9) PBS with 5 ng/ml VLY and 1000 ng/ml antibody 23A2.

In summary, all these data indicate that monoclonal antibodies 9B4 and 23A2 are specific to vaginolysin (VLY) from bacteria *Gardnerella vaginalis* and neutralize its cytolytic activity. Therefore, these monoclonal antibodies can be used to detect VLY by imunochemical assays for the diagnosis of *Gardnerella vaginalis* infection. For instance, by using these antibodies it is possible to test cytological specimens for the presence of VLY by an immunohistochemical assay, ELISA or Western blotting. If the VLY test is positive, this confirms *Gardnerella vaginalis* infection.

Newly developed monoclonal antibodies 9B4 and 23A2 have a potent neutralizing activity, therefore, these antibodies or their recombinant analogs can be applied for prophylactic and therapeutic purposes for treatment of pathology induced by *Gardnerella vaginalis* infection. For instance, by using genetic material (RNA) from hybridomas 9B4 and 23A2 single-chain recombinant antibody fragments (scFv) or humanized antibodies can be constructed that should neutralize biological activity of VLY. As VLY is the main virulence factor of *Gardnerella vaginalis,* its neutralization could be an efficient way to treat infection-induced pathology.

## Claims

1. A hybridoma cell line 9B4, deposited as DSM ACC2991.

2. A hybridoma cell line 23A2, deposited as DSM ACC2992.

3. A hybridoma cell line, selected from a group comprising 9B4, deposited as DSM ACC2991 and 23A2, deposited as DSM ACC2992, producing monoclonal antibody neutralizing cytolytic activity of vaginolysin.

4. A monoclonal antibody, that recognizes amino acid sequence SEQ ID: NO.1 or similar sequence, which identity is at least 95%,
produced by hybridoma cell line as defined in any of Claims 1-3.

5. Monoclonal antibody, produced by the hybridoma cell line according to any one of Claims 1-3, which is an IgG antibody.

6. Monoclonal antibody according to Claim 5, which is a murine monoclonal antibody.

7. Use of the monoclonal antibody according to Claim 4 or Claim 5 for producing of preparations, comprising said monoclonal antibody, for detection of infection, such as bacterial vaginosis induced by *Gardnerella vaginalis.*

8. A diagnostic kit for the detection of *Gardnerella vaginalis* infection, comprising at least a monoclonal antibody according to Claim 4 or Claim 5.

9. Use of the hybridoma according to any of Claims 1-3 for manufacture of preparations, comprising monoclonal antibodies, in the method for inhibiting or neutralizing cytolitic activity of vaginolysin.

10. A preparation, comprising monoclonal antibodies, produced from hybridoma according to any of Claims 1-3 for inhibiting of infection, such as bacterial vaginosis induced by *Gardnerella vaginalis.*

11. A pharmaceutical composition, comprising an effective amount of preparation according to Claim 10 in combination with pharmaceutically acceptable carrier, diluent, excipient and/or auxiliary substances for the prophylaxis and/or treatment of conditions induced by *Gardnerella vaginalis* infection.

## Patentansprüche

1. Eine Hybridomazellinie 9B4, hinterlegt als DSM ACC2991 hinterlegt.

2. Eine Hybridomazellinie 23A2, hinterlegt als DSM ACC2992 hinterlegt.

3. Eine Hybridomazellinie, ausgewählt aus einer Gruppe, 9B4, hinterlegt als DSM ACC2991 und 23A2 abgeschieden, wie DSM ACC2992 hinterlegt, die monoklonale Antikörper neutralisierende zytolytische Aktivität vaginolysin.

4. Ein monoklonaler Antikörper, der eine Aminosäuresequenz SEQ 10 erkennt: NO.1 oder ähnliche Sequenz, die Identität zumindest 95%,
durch Hybridom-Zellinie nach irgendeinem der Ansprüche 1-3 hergestellt wird.

5. Monoklonaler Antikörper, der von dem Hybridom-Zelllinie nach einem der Ansprüche 1-3, die ein IgG-Antikörper ist, produziert.

6. Monoklonale Antikörper nach Anspruch 5, der ein muriner monoklonaler Antikörper ist.

7. Verwendung der monoklonalen Antikörper nach Anspruch 4 oder Anspruch 5 zur Herstellung von Zubereitungen, enthaltend den monoklonalen Antikörper, der zum Nachweis von Infektionen, wie bakterielle Vaginose von *Gardnerella vaginalis* induziert.

8. Diagnostischer Kit zum Nachweis von *Gardnerella vaginalis* Infektion, umfassend wenigstens einen monoklonalen Antikörper nach Anspruch 4 oder Anspruch 5.

9. Verwendung des Hybridoms nach einem der Ansprüche 1-3 zur Herstellung von Präparaten, die aus monoklonalen Antikörpern, die in dem Verfahren zur Hemmung oder Neutralisierung cytolitic Aktivität vaginolysin.

10. Eine Präparation, umfassend monoklonale Antikörper, erhalten von Hybridom nach einem der Ansprüche 1-3 zur Inhibierung der Infektion hergestellt, wie bakterielle Vaginose von *Gardnerella vaginalis* induziert.

11. Eine pharmazeutische Zusammensetzung, die eine wirksame Menge der Zubereitung nach Anspruch 10 in Kombination mit pharmazeutisch verträglichen Träger, Verdünnungsmittel, Hilfsstoff und / oder Hilfssubstanzen für die Prophylaxe und / oder Behandlung von durch *Gardnerella vaginalis* Infektion induziert Anspruch.

## Revendications

1. Une ligne de cellules d'hybridome 9B4, déposé sous le numéro DSM ACC2991.

2. Lignée cellulaire d'hybridome 23a2, déposé sous le numéro DSM ACC2992.

3. Une lignée cellulaire d'hybridomes, choisis parmi un groupe comprenant 9B4, déposé sous le numéro DSM ACC2991 et 23a2, déposé sous le numéro DSM ACC2992, produisant un anticorps monoclonal neutralisant l'activité cytolytique des vaginolysin.

4. Un anticorps monoclonal, qui reconnaît la séquence d'acides aminés SEQ ID: N° 1 ou une séquence similaire, qui est une identité d'au moins 95%,
produit par la lignée cellulaire d'hybridome selon l'une quelconque des revendications 1-3.

5. Anticorps monoclonal, produit par la lignée cellulaire d'hybridome selon l'une quelconque des revendications 1 à 3, qui est un anticorps IgG.

6. Anticorps monoclonal selon la revendication 5, qui est un anticorps monoclonal murin.

7. Utilisation de l'anticorps monoclonal selon la revendication 4 ou la revendication 5 pour la fabrication de préparations, comprenant ledit anticorps monoclonal, pour la détection de l'infection, tels que la vaginose bactérienne induite par *Gardnerella vaginalis.*

8. Un kit de diagnostic pour la détection de l'infection *Gardnerella vaginalis,* comprenant au moins un anticorps monoclonal selon la revendication 4 ou la revendication 5.

9. Utilisation de l'hybridome selon l'une quelconque des revendications 1-3 pour la fabrication de préparations, comprenant des anticorps monoclonaux, dans le procédé pour l'inhibition de l'activité cytolytique ou de neutralisation de vaginolysin.

10. Une préparation comprenant des anticorps monoclonaux, produits à partir d'hybridomes selon l'une quelconque des revendications 1-3 pour l'inhibition de l'infection, telles que la vaginose bactérienne induite par *Gardnerella vaginalis.*

11. Composition pharmaceutique, comprenant une quantité efficace de préparation selon la revendication 10 en combinaison avec un support pharmaceutiquement acceptable, diluant, excipient et / ou des substances auxiliaires pour la prophylaxie et / ou le traitement d'affections induites par une infection *Gardnerella vaginalis.*


### SEQUENCE LISTING

<110> BIOTECHNOLOGIJOS INSTITUTAS UAB PROFARMA
<120> MONOCLONAL ANTIBODIES AGAINST VAGINOLYSIN
<130> 1690PCT
<150> LT2009012
   <151> 2009-02-23
<160> 1
   <170> PatentIn version 3.3
<210> 1
   <211> 516
   <212> PRT
   <213> Gardnerella vaginalis
<400> 1
   Met Asn Asn Thr Lys Phe Tyr Arg Asn Ala Ala Met Leu Leu Leu Ala
   1               5                   10                  15
   Gly Ala Thr Ile Ile Pro Gln Cys Leu Ala Ala Pro Ala Met Ala Ala
               20                  25                  30
   Pro Ala Ala Lys Asp Ser Glu Pro Thr Ala Ser Cys Ala Ala Lys Lys
           35                  40                  45
   Asp Ser Leu Asn Asn Tyr Leu Trp Asp Leu Gln Tyr Asp Lys Thr Asn
       50                  55                  60
   Ile Leu Ala Arg His Gly Glu Thr Ile Asp Asn Lys Phe Ser Ser Asp
   65                  70                  75                  80
   Ser Phe Asn Lys Gly Asp Glu Phe Val Val Val Glu His Gln Lys Lys
                   85                  90                  95
   Asn Ile Thr Asn Thr Thr Ser Asn Leu Ser Val Thr Ser Ala Asn Asp
               100                 105                 110
   Asp Arg Val Tyr Pro Gly Ala Leu Phe Arg Ala Asp Gln Asn Leu Met
           115                 120                 125
   Asp Asn Met Pro Ser Leu Ile Ser Ala Asn Arg Ala Pro Ile Thr Leu
       130                 135                 140
   Ser Val Asp Leu Pro Gly Phe His Gly Gly Glu Ser Ala Val Thr Val
   145                 150                 155                 160
   Gln Arg Pro Thr Lys Ser Ser Val Thr Ser Ala Val Asn Gly Leu Val
                   165                 170                 175
   Ser Lys Trp Asn Ala Gln Tyr Ala Ala Ser His His Val Ala Ala Arg
               180                 185                 190
   Met Gln Tyr Asp Ser Ala Ser Ala Gln Ser Met Ser Gln Leu Lys Ala
           195                 200                 205
   Lys Phe Gly Ala Asp Phe Ala Lys Ile Gly Val Pro Leu Lys Ile Asp
       210                 215                 220
   Phe Asp Ala Val His Lys Gly Glu Lys Gln Thr Gln Ile Val Asn Phe
   225                 230                 235                 240
   Lys Gln Thr Tyr Tyr Thr Val Ser Val Asp Ala Pro Asp Ser Pro Ala
                   245                 250                 255
   Asp Phe Phe Ala Pro Cys Thr Thr Pro Glu Ser Leu Lys Ser Arg Gly
               260                 265                 270
   Val Asp Ser Lys Arg Pro Pro Val Tyr Val Ser Asn Val Ala Tyr Gly
           275                 280                 285
   Arg Ser Met Tyr Val Lys Phe Asp Thr Arg Ser Lys Ser Thr Asp Phe
       290                 295                 300
   Gln Ala Ala Val Glu Ala Ala Ile Lys Gly Val Glu Ile Lys Pro Asn
   305                 310                 315                 320
   Thr Glu Phe His Arg Ile Leu Gln Asn Thr Ser Val Thr Ala Val Ile
                   325                 330                 335
   Leu Gly Gly Ser Ala Asn Gly Ala Ala Lys Val Ile Thr Gly Asn Val
               340                 345                 350
   Asp Thr Leu Lys Ala Leu Ile Gln Glu Gly Ala Asn Leu Ser Thr Ser
           355                 360                 365
   Ser Pro Ala Val Pro Ile Ala Tyr Thr Thr Ser Phe Val Lys Asp Asn
       370                 375                 380
   Glu Val Ala Thr Leu Gln Thr Asn Ser Asp Tyr Val Glu Thr Lys Val
   385                 390                 395                 400
   Ser Ser Tyr Arg Asp Gly Tyr Leu Thr Leu Asp His Arg Gly Ala Tyr
                   405                 410                 415
   Val Ala Arg Tyr Tyr Ile Tyr Trp Asp Glu Tyr Gly Thr Glu Ile Asp
               420                 425                 430
   Gly Thr Pro Tyr Val Arg Ser Arg Ala Trp Glu Gly Asn Gly Lys Tyr
           435                 440                 445
   Arg Thr Ala His Phe Asn Thr Thr Ile Gln Phe Lys Gly Asn Val His
       450                 455                 460
   Asn Leu Arg Ile Lys Leu Val Glu Lys Thr Gly Leu Val Trp Glu Pro
   465                 470                 475                 480
   Trp Arg Thr Val Tyr Asp Arg Ser Asp Leu Pro Leu Val Arg Gln Arg
                   485                 490                 495
   Thr Ile Lys Asn Trp Gly Thr Thr Leu Trp Pro Arg Val Ala Glu Thr
               500                 505                 510
   Val Lys Asn Asp
           515